(19) Europäisches Patentamt

European Patent Office

Office européen des brevets

(11) Veröffentlichungsnummer: **0 294 672 B1**

(12) **EUROPÄISCHE PATENTSCHRIFT**

(45) Veröffentlichungstag der Patentschrift: **12.08.92**

(51) Int. Cl.⁵: **B65D 81/32**, B05C 17/00, A61M 5/19

(21) Anmeldenummer: **88108552.6**

(22) Anmeldetag: **28.05.88**

(54) **Doppel-Austragkartusche für Zweikomponentenmassen.**

(30) Priorität: **10.06.87 CH 2176/87**

(43) Veröffentlichungstag der Anmeldung:
**14.12.88 Patentblatt 88/50**

(45) Bekanntmachung des Hinweises auf die
Patenterteilung:
**12.08.92 Patentblatt 92/33**

(84) Benannte Vertragsstaaten:
**CH DE ES FR GB IT LI NL SE**

(56) Entgegenhaltungen:
EP-A- 0 156 098     EP-A- 0 236 129
DE-A- 3 420 323     DE-A- 3 501 331
FR-A- 2 399 861     GB-A- 839 285
US-A- 3 782 600     US-A- 4 260 077

(73) Patentinhaber: **Keller, Wilhelm A.**
**Grundstrasse 12**
**CH-6343 Rotkreuz(CH)**

(72) Erfinder: **Keller, Wilhelm A.**
**Grundstrasse 12**
**CH-6343 Rotkreuz(CH)**

(74) Vertreter: **Kägi, Otto**
**Patentanwalt Hinterbergstrasse 36 Postfach**
**CH-6330 Cham(CH)**

## Beschreibung

Die Erfindung betrifft eine Doppel-Austragkartusche, die für die getrennte Langzeitlagerung und Verteilung von Zweikomponentenmassen von deren Abfüllung in die Doppel-Austragkartusche bis zur Verwendung durch den Verbraucher, sowie zur Bedienung der Kartusche mittels eines Austraggerätes beim Verbraucher bestimmt ist, mit zwei doppelwandig getrennten, miteinander verbundenen und parallelachsig nebeneinanderliegenden Vorratszylindern, die je eine stirnseitige Austrittsöffnung aufweisen, wobei beide Austrittsöffnungen einander benachbart auf der gemeinsamen Durchmesserlinie beider Vorratszylinder liegen und wobei der Achsabstand der Austrittsöffnungen kleiner ist als der Achsabstand der beiden Vorratszylinder.

Zweikomponenten-Systeme, z.B. für Kleber, Dichtmassen, Dental-Abdruckmassen usw. bestehen aus zwei unterschiedlichen, zusammengehörigen Stoffkomponenten, die in bekannter Weise in getrennten Vorratszylindern gelagert und vertrieben werden. Zur Verarbeitung werden die beiden Massen in bestimmtem Verhältnis vermischt, worauf sie chemisch reagieren und aushärten bzw. sich verfestigen. Das Austragen der beiden Komponenten aus den Zylindern erfolgt durch gleichzeitiges Betätigen von in jedem Zylinder angeordneten Förderkolben, wodurch die Massen durch die stirnseitige Austrittsöffnung ausgepresst werden.

Eine Doppel-Austragkartusche der oben genannten Art ist aus der US-A-4 260 077 bekannt - zwar für Handbetätigung, obgleich eine Bedienung mittels Austraggerät nicht ausgeschlossen erscheint.

Dort sind die Austragsöffnungen beider Zylinder je mit einer Düse versehen, so dass zwei getrennte Materialstränge ausgepresst werden, die dann von Hand vermischt werden müssen. Im übrigen ist jene Doppelkartusche speziell so gestaltet, dass ihre Zylinder getrennt und auch einzeln verwendet werden können. Das nachträgliche Mischen der Komponenten von Hand ist indessen unrationell, und eine gleichmässige Durchmischung, wie sie für einwandfreie Ergebnisse unerlässlich ist, kann nicht gewährleistet werden. Für ein bequemes und intensives Vermischen der Komponenten ist es deshalb üblich, diese durch ein an die Kartuschenzylinder angeschlossenes Mischerrohr hindurchzupressen (sogenannter statischer oder Strömungsmischer).

Zum Anschluss von Zweikomponenten-Kartuschen an ein Mischerrohr ist es bekannt, besondere Verbindungsstücke zu verwenden; diese können Bestandteil eines Kartuschen-Bedienungsgerätes sein (z.B. nach DE-A-35 01 331) oder von einem solchen Gerät unabhängig sein (DE-A-34 20 323, Fig. 4 und EP-A-0 236 129, Fig. 2). Solche Anordnungen sind allerdings mit verschiedenen und erheblichen Nachteilen behaftet:

- Es können nur Einzelkartuschen verwendet werden, so dass bei der Anwendung Verwechslungen nicht auszuschliessen sind (irrtümliches Zusammenführen von zwei nicht zusammengehörigen bzw. von zwei gleichen Komponenten).
- Das Zusammenfügen der Einzelkartuschen, des Verbindungsstücks und des Mischerrohres kann erst unmittelbar vor der Anwendung erfolgen, was für jede neue Kartusche umständliche Vorbereitungen bedeutet.
- Die Einzelkartuschen sind mit einem stirnseitigen Gewindestutzen versehen, welcher in je ein Innengewinde am Verbindungsstück eingedreht wird. Eine Verbindung zwischen den Einzelkartuschen ist deshalb nicht möglich, weshalb die Anordnung mechanisch wenig stabil ist; ferner müssen die Gewindestutzen notwendigerweise zentrisch, d.h. zur Zylinderachse koaxial sein.
- Der letztgenannte Umstand führt am Verbindungsstück zu einem grossen Abstand zwischen den beiden Eintrittsstellen (Innengewinde), wodurch sich lange, abgewinkelte Leitungskanäle von dort zum Mischeranschluss ergeben, die schwierig herzustellen sind und ein erhebliches "totes Volumen" aufweisen. Solche Verbindungsstücke sind deshalb kaum als billige Wegwerfteile herzustellen, wodurch wiederum Probleme mit der Reinigung usw. entstehen.

Eine weitere bekannte Anordnung (FR-A-2 399 861) geht ebenfalls von Einzelkartuschen aus. Als Verbindungsstück dient eine Art dreischenkliges Rohr, wobei im einen Schenkel eine Mischeinrichtung integriert ist und die beiden andern Schenkel auf Austrittsstutzen an den Kartuschen aufgesteckt werden sollen. Ein solches Teil ist - wenn überhaupt - höchstens als gummielastischer Schlauch herstellbar. Ein solcher kann jedoch keine formstabile Verbindung zwischen den Einzelkartuschen schaffen und der Anschluss an den Stutzen kann dem hohen Druck, welcher beim Auspressen der Komponenten auftritt, kaum standhalten.

Aufgabe der vorliegenden Erfindung ist es, die erwähnten Nachteile bekannter Produkte zu beseitigen und eine gebrauchsfertige, stabile Doppelkartusche zu schaffen, welche die einwandfreie Verteilung und Langzeit-Lagerung beider zusammengehöriger Komponenten ab Herstell- bzw. Abfüllwerk ermöglicht und an die bei Gebrauch das Mischerrohr ohne Umstände direkt anschliessbar ist.

Die erfindungsgemässe Doppel-Austragkartusche ist dadurch gekennzeichnet, dass ein den Vorratszylindern vorgelagerter Uebergangsteil als Bestandteil der Doppel-Austragkartusche vorgesehen ist, welcher eine für beide Komponenten gemeinsame Mündung zum Anschluss eines Mischerrohres aufweist und zwei bis an das Ende der Mündung getrennte Austragkanäle enthält, und dass der Uebergangsteil über eine permanente axiale Steckverbindung, welche beide Austragkanäle je an die Austrittsöffnung der Vorratszylinder anschliesst, mit beiden Vorratszylindern verbunden ist, wodurch der Uebergangsteil und beide Vorratszylinder zusammen eine starre, zur getrennten Langzeitlagerung beider Komponenten bestimmte Einheit bilden.

Dank dieser Gestaltung bietet die Herstellung des Uebergangsteils wie auch der Zylinder als einfache Wegwerfteile kaum Schwierigkeiten. Vor allem kann auch eine sehr erwünschte, weitgehende Standardisierung der Teile erreicht werden im Hinblick auf Doppel-Austragkartuschen mit verschiedenen Querschnittsverhältnissen ihrer Zylinder, wie sie für verschiedene Mischungsverhältnisse der Komponenten benötigt werden.

Verschiedene sich ergebende, zweckmässige Ausgestaltungen des Erfindungsgegenstandes sind in den Patentansprüchen 2 bis 17 angegeben; insbesondere können die beiden zusammengehörigen Vorratszylinder entweder einstückig (zusammenhängend) hergestellt werden (Ansprüche 7 und 8) oder aber getrennt (Anspruch 9), wobei dann für die Zylinder eines Paares verschiedene Werkstoffe gewählt werden können, sei es aus Festigkeitsgründen oder wegen der Verträglichkeit mit dem aufzunehmenden Inhalt.

Ausgehend von den Doppel-Austragkartuschen gemäss den bereits genannten Ansprüchen beziehen sich die Ansprüche 16 und 17 schliesslich auf eine Baureihe solcher Doppelkartuschen mit gleichem Zylinder-Achsabstand und gleichem Gesamt-Nennvolumen, aber unterschiedlichen Querschnittsverhältnissen ihrer Zylinderpaare; die so definierte Baureihe ist besonders geeignet zur Bedienung durch immer dasselbe Austraggerät mit gegebenem, mit dem Zylinder-Achsabstand übereinstimmendem Abstand der Austragstössel.

Nachstehend werden Ausführungsbeispiele der Erfindung im Zusammenhang mit der Zeichnung näher erläutert.

Fig.1 ist eine Seitenansicht einer Doppelkartuschen-Einheit, bestehend aus einem Paar von Vorratszylindern und mit diesem permanent verbundenem Uebergangsteil; ebenfalls dargestellt ist ein anzuschliessendes Mischerrohr und ein wahlweise einzusetzender Verschlusspfropfen;

Fig. 2 ist eine Stirnansicht der Doppelkartusche nach Fig. 1 ohne Uebergangsteil;

Fig. 3 zeigt einen Längsschnitt durch die Doppel-Austragkartusche nach Fig.1 vor dem Herstellen der Steckverbindung zwischen den Vorratszylindern und dem Uebergangsteil, wobei auch mehrere einer Baureihe zugehörige Zylinderpaare mit verschiedenen Querschnittsverhältnissen angedeutet sind;

Fig. 4 ist ein Schnitt entlang der Linie IV - IV in Fig. 3 bei aufgesetztem Uebergangsteil;

Fig. 5 ist eine teilweise Frontansicht der Doppelkartusche mit Uebergangsteil nach Fig. 3;

Fig. 6 ist eine teilweise Stirnansicht des hinteren Endes der Doppelkartusche in Richtung des Pfeiles VI in Fig. 3; und

Fig. 7 zeigt als Stirnansicht ein Beispiel einer distanzhaltenden Schnappverbindung der beiden Vorratszylinder, wenn diese getrennt hergestellt sind.

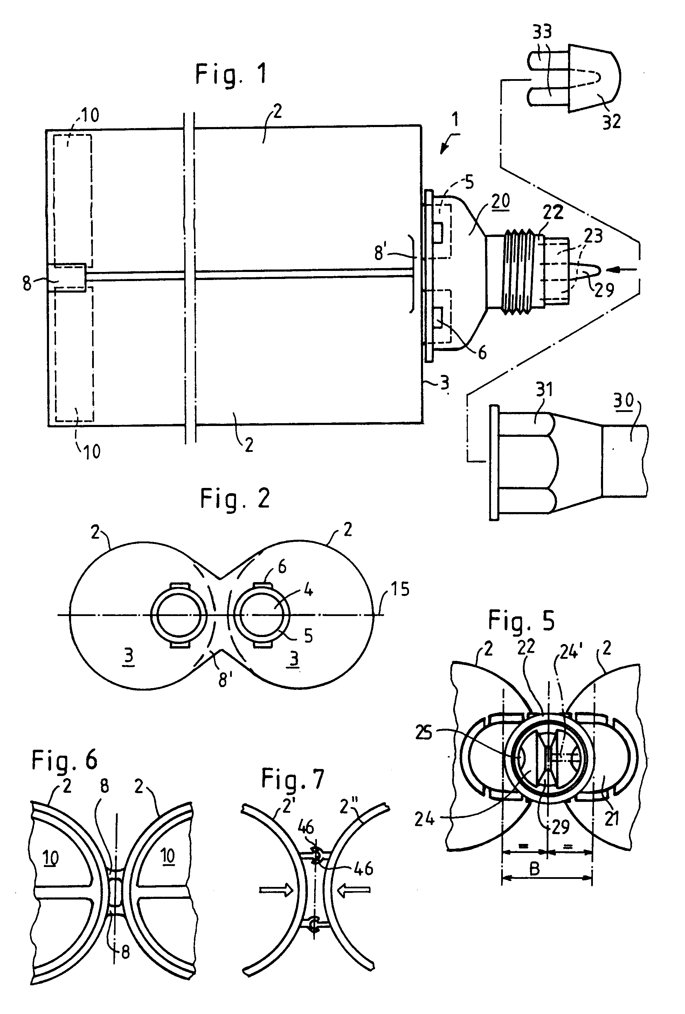

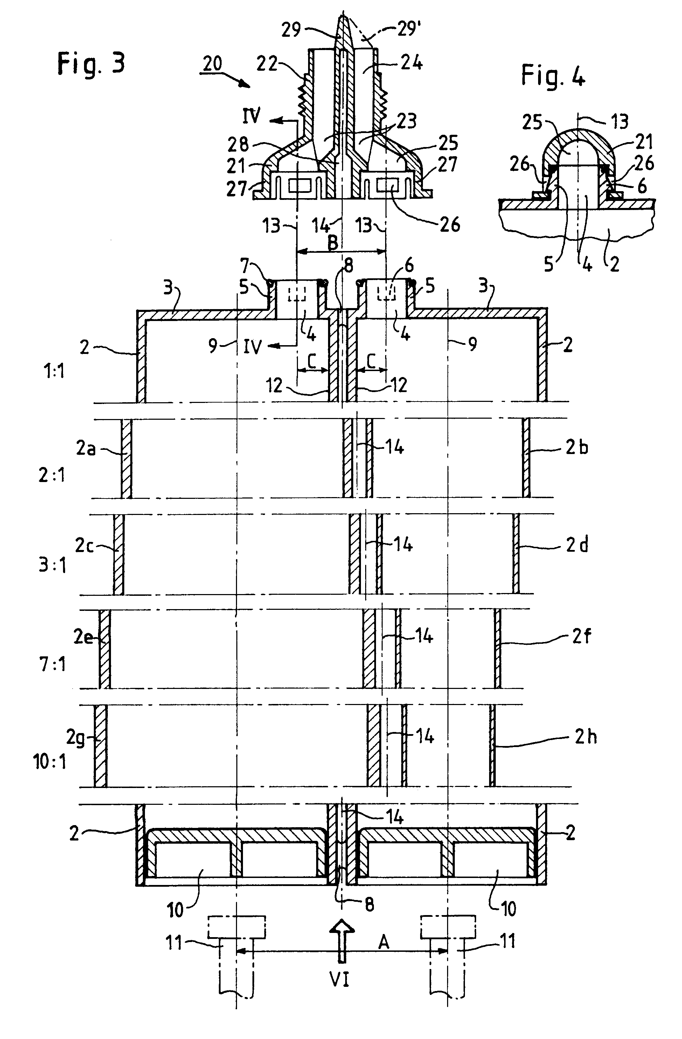

Die in den Fig. 1 bis 6 dargestellte Doppel-Austragkartusche 1 für Zweikomponentenmassen setzt sich zusammen aus zwei parallelachsig nebeneinander angeordneten, doppelwandig getrennten Vorratszylindern 2 und einem beiden Vorratszylindern vorgelagerten Uebergangsteil 20. In das hintere Ende jedes Zylinders (links in Fig. 1 und unten in Fig. 3) ist ein Förderkolben 10 eingesetzt. Die beiden Vorratszylinder 2 sind dazu bestimmt, je eine Masse bzw. Komponente eines sogenannten Zweikomponentensystems aufzunehmen. Zur Verwendung wird die Austragkartusche in an sich bekannter Weise in ein Austraggerät eingesetzt, welches zwei parallele Stössel 11 aufweist, die in Fig. 3 unten strichpunktiert angedeutet sind. Bei Bedienung des im übrigen nicht dargestellten Austraggerätes werden dessen Stössel 11 synchron gegen die beiden Förderkolben 10 bewegt, und durch den Vortrieb der Förderkolben innerhalb der Vorratszylinder werden die Zylinderinhalte durch je eine stirnseitige Austrittsöffnung 4 dosiert ausgetragen.

An beiden miteinander verbundenen Vorratszylindern 2 sind die stirnseitigen Austrittsöffnungen 4, wie aus Fig. 2 hervorgeht, einander benachbart (exzentrisch zu der jeweiligen Zylinderachse) und auf der beiden Zylindern gemeinsamen Durchmesserlinie 15 gelegen. Gemäss Fig. 1 sind beide Zylinder 2 und der Uebergangsteil 20 zu einer starren Einheit 1 verbunden, und zwar mittels einer permanenten axialen Steckverbindung, wie sie weiter unten näher beschrieben ist. Durch die Steckverbindung besteht auch ein dichter Anschluss zwischen den Austrittsöffnungen 4 und zwei zugehörigen Austragkanälen 23, die im Uebergangsteil 20 bis zum Ende einer Mündung 22 getrennt verlaufen. Die Mündung 22 - gemäss Fig. 5 mit kreisförmigem Umfang - ist somit beiden Komponenten gemeinsam. Sie dient bei der Verarbeitung des Kartuscheninhalts für den Anschluss eines Mischerrohres 30, vorzugsweise eines statischen Mischers (in Fig. 1 abgebrochen dargestellt). Der Anschluss am Aussengewinde der Mündung 22 erfolgt in bekannter Weise mittels einer am Rohr 30 angeformten Gewindehülse 31 oder mit Hilfe einer Ueberwurfmutter. Eine über die Mündung 22 vorstehende Trennwand 29 hält die beiden Komponenten bis zum ersten Mischelement (nicht sichtbar) im Mischerrohr 30 getrennt.

Während der Lagerung und Verteilung der gefüllten Doppelkartusche 1 kann die Mündung 22 durch einen Pfropfen 32 mit in die Kanäle 23 ragenden Zapfen 33 verschlossen sein; der Pfropfen kann dabei durch die vorgenannte Ueberwurfmutter (nicht dargestellt) festgehalten werden. Eine andere Möglichkeit des Verschlusses besteht darin, in oder hinter den Oeffnungen 4 sogenannte Berstfolien oder -scheiben anzubringen, welche bei beginnendem Materialaustrag durch den Innendruck in den Zylindern 2 zum Platzen gebracht werden.

Die beiden Vorratszylinder 2 sind beim vorliegenden Ausführungsbeispiel aus Kunststoff einstückig im Spritzgiessverfahren hergestellt, und zwar so, dass sie voneinander doppelwandig getrennt sind. Der Zusammenhang zwischen beiden Zylindern ist z.B. durch je zwei Stege 8 im Bereich des hinteren (Fig. 6) und vorderen Zylinderendes hergestellt (Fig. 3); anstelle der vorderen Stege kann auch eine mit den Stirnseiten 3 bündige "Brücke" 8' gemäss Fig. 1 und 2 die Verbindung herstellen.

Die Austrittsöffnung 4 in jeder Zylinderstirnseite 3 ist durch eine runde Bohrung gebildet, deren Achse 13 vorzugsweise bei beiden Zylindern den gleichen Abstand C zur Zylinder-Innenwand 12 aufweist. Beide Austrittsöffnungen 4 sind von je einem kreisrunden, von der jeweiligen Stirnseite 3 ausgehenden und vorzugsweise zylindrischen Stutzen 5 umgeben.

Der dem Zylinderpaar 2 zugeordnete Uebergangsteil 20 ist ebenfalls als Kunststoff-Spritzgussteil hergestellt. Er weist eine den Zylindern zugekehrte Anschlusspartie 21 und die genannte Mündung 22 auf. In der Anschlusspartie 21 sind zwei im wesentlichen zylindrische Hülsen 27 geformt, mit welchen sie die beiden Stutzen 5 passend übergreift. Die Form der zwei im Uebergangsteil 20 enthaltenen, getrennten Austragkanäle 23 geht aus den Figuren 3 und 5 deutlich hervor. Hauptsächlich aus spritzgiesstechnischen Gründen ist jeder Austragkanal 23 wie ersichtlich vorzugsweise durch zwei Längsabschnitte 24 und 25 gebildet, die zueinander parallelachsig versetzt sind, sich jedoch im Querschnitt überschneiden. Wie die beiden Zylinder 2 sind auch die Kanäle Z3 zweckmässigerweise auf ihrer ganzen Länge, d.h. bis zum Ende der Mündung 22 doppelwandig durch einen Hohlraum 28 getrennt. Durch die doppelwandige Trennung wird gewährleistet, dass zwischen den beiden eingefüllten Komponentenmassen auch bei längerer Lagerung keine Diffusion bzw. vorzeitige chemische Reaktion stattfindet.

Am Uebergangsteil 20 ist die Anschlusspartie 21 vorzugsweise symmetrisch zur Achse 14 der gemeinsamen Mündung 22 gestaltet. Bei gleichem Abstand C der Stutzenachsen 13 von der jeweiligen Zylinder-Innenwand 12 fällt dann die Achse 14 in die Mitte zwischen diesen beiden Innenwänden (Fig. 3).

Wenn es sich um eine Doppel-Austragkartusche handelt, bei der die Querschnitte der beiden Zylinder 2 gleich sind, werden die Austrittsöffnungen 4 wie auch die Austragkanäle 23 im Uebergangsteil mit gleichem Querschnitt ausgeführt. Sind jedoch - für ein von 1:1 abweichendes Volumen-Mischungsverhältnis der beiden Komponenten - die beiden Zylinder-Querschnitte des Zylinderpaares verschieden, so können die Oeffnungen 4 und die anschliessenden Längsabschnitte 25 dennoch gleich (und symmetrisch zur Achse 14) ausgeführt werden, hingegen ist es zweckmässig, die Ausgangs-Querschnitte der beiden Austragkanäle 23 in der gemeinsamen Mündung 22 im gleichen Verhältnis zueinander zu bemessen wie die Querschnitte der beiden Vorratszylinder; in Fig. 5 ist auf der rechten Seite strichpunktiert ein entsprechend verengter vorderer Kanalabschnitt 24' (verglichen mit dem Kanalabschnitt 24 links in der Figur) angedeutet. Wie in Fig. 3 schematisch bei 29' eingetragen, ist dann auch die Wand 29 für den Anschluss an den Mischer entsprechend zu gestalten. Diese Querschnitts-Anpassung am Ausgang der Austragkanäle 23 bewirkt, dass bei ungleichem Mischungsverhältnis die beiden Komponenten zwar mit entsprechend verschiedenem Volumenverhältnis, jedoch in gleich langen Strängen und mit gleicher Geschwindigkeit in den Mischer übertreten.

Die permanente, axiale Steckverbindung des Uebergangsteils 20 mit den beiden Vorratszylindern 2 kann gemäss Fig. 3 und 4 durch einen Schnappverschluss hergestellt sein: Beispielsweise an der Aussenseite jedes Stutzens 5 ist an zwei gegenüberliegenden Stellen je eine keilförmige Nase 6 angeformt. An den entsprechenden Stellen der Hülsen 27 am Uebergangsteil befindet sich ein rechteckiges "Fenster" 26; die Hülsenwand kann zu beiden Seiten jedes Fensters 26 wie dargestellt eingeschnitten sein, so dass der betreffende Wandabschnitt etwas ausbiegbar ist. Wird die Anschlusspartie 21 über die beiden Stutzen 5 geschoben, so schnappen die Nasen 6 in die Fenster 26 ein, wodurch eine solide Verankerung zwischen beiden Vorratszylindern und dem Uebergangsteil zustandekommt.

Gemäss Fig. 3 und 4 ist jeder Stutzen 5 zur Anschlusspartie 21 durch einen O-Ring 7 abgedichtet; der Dichtungsring 7 kann wie dargestellt im Stirnbereich oder in einer Umfangsnut des Stutzens 5 angeordnet sein. Anstelle eines Dichtungsringes 7 könnte auch eine Lippendichtung zwischen jedem Stutzen 5 und der Anschlusspartie 21 vorgesehen sein (nicht dargestellt).

Als Steckverbindung zwischen den Zylindern und dem Uebergangsteil sind selbstverständlich andere Varianten von Schnappverbindungen denkbar, die Steckverbindung kann auch z.B. durch Kleben oder Schweissen gesichert sein. In jedem Fall ist jedoch die Steckverbindung permanent, d.h. nicht zum nachträglichen Lösen bestimmt; die aus den Bestandteilen 2 und 20 zusammengefügte Einheit 1 bildet als Ganzes ein Wegwerfteil. Eine Schnappverbindung der beschriebenen Art oder eine wie oben erwähnt "gesicherte" Steckverbindung kann auch nicht (im Gegensatz zu einer Verschraubung) durch Drehen der Zylinder gelöst werden. Zur Formsteifheit der Einheit 1 trägt auch der Umstand wesentlich bei, dass beide Zylinder 2 untereinander direkt verbunden sind.

Es kann zweckmässig sein, die beiden Vorratszylinder eines Paares getrennt herzustellen und dann zusammenzufügen. Sie können dann insbesondere aus verschiedenen Werkstoffen gefertigt werden, beispielsweise aus Festigkeitsgründen oder im Hinblick auf die Beständigkeit bzw. Verträglichkeit mit dem aufzunehmenden Inhalt. Getrennt hergestellte Zylinder sollen mindestens im Bereich ihres hinteren Endes durch eine distanzhaltende Verbindung zusammengehalten werden. Eine ebensolche Verbindung kann auch im Bereich des vorderen Zylinderendes vorgesehen sein, es ist aber auch denkbar, dass die Zylinder vorne nur durch den Uebergangsteil 20 zusammengehalten werden. Vorzugsweise an ihrem vorderen Ende sollen die Zylinder eine Sicherung gegen Längsverschiebung aufweisen, z.B. mittels einander übergreifender Lappen. Ein Beispiel einer geeigneten distanzhaltenden Schnappverbindung ist in Fig. 7 dargestellt: An zwei getrennt hergestellten Zylindern 2' und 2" sind je zwei identische und symmetrische Profile 46 angeformt, welche in Pfeilrichtung radial ineinandergedrückt werden können und eine stabile Schnappverbindung herstellen. Auch eine axial aufsteckbare, die Zylinder umfassende "Brille" ist als distanzhaltende Verbindung denkbar.

Der beschriebene Aufbau von Doppel-Austragkartuschen mit stirnseitig vorgelagertem Uebergangsteil ermöglicht eine relativ einfache, rationelle Fertigung und Montage. Die Einheit weist eine gute Stabilität im Gebrauch auf und gewährleistet völlige Dichtheit und einwandfreie Trennung der beiden Komponenten auch bei längerer Lagerzeit. Das Mischerrohr kann ohne Umstände an die gemeinsame Mündung der gebrauchsfertigen Einheit angeschlossen werden.

Vor allem ermöglicht diese Bauweise aber auch, eine weitgehend standardisierte Baureihe von mehreren Doppel-Austragkartuschen mit gleichem Gesamt-Nennvolumen zu bilden, wobei von Doppelkartusche zu Doppelkartusche das Verhältnis der Querschnitte des Zylinderpaars verschieden ist. Es können so Doppelkartuschen für Austrag- bzw. Mischungsverhältnisse der Komponenten von beispielsweise 1:1, 2:1 usw. bis beispielsweise 10:1, aber auch je nach Erfordernis für nicht ganzzahlige Verhältnisse gebildet werden. In Fig. 3 sind für verschiedene von 1:1 abweichende Querschnitts- bzw. Mischungsverhältnisse die entsprechenden Zylinderdurchmesser der Zylinderpaare 2a, 2b; 2c, 2d; 2e, 2f; 2g, 2h einer solchen Baureihe dargestellt (bei gegebenem Gesamt-Nennvolumen beider Zylinder und jeweils gleich vorausgesetzter Baulänge). Wie ersichtlich, können entsprechend den Festigkeitsanforderungen auch die Wandstärken den verschiedenen Zylinderdurchmessern angepasst werden. Ein besonderes Merkmal einer solchen Baureihe besteht darin, dass bei allen Zylinderpaaren der Reihe der Zylinder-Achsabstand A gleich ist und mit dem gegebenen Achsabstand der Stössel 11 eines zugeordneten Austraggerätes übereinstimmt. Dank diesem Merkmal ist es möglich, alle Kartuschen der Baureihe mit dem gleichen Austraggerät zu bedienen, wobei die Stösselkräfte immer zentrisch auf die jeweiligen Förderkolben 10 übertragen werden.

Bei der Gestaltung der Baureihe ist es überdies möglich, bei allen Zylinderpaaren den Abstand B zwischen den Achsen 13 der beiden Stutzen gleich zu halten und dadurch im wesentlichen immer gleiche Uebergangsteile 20 zu verwenden. Wie aus Fig. 3 hervorgeht, verschiebt sich dabei lediglich die Lage der Symmetrieachse 14 zwischen den Zylinderachsen 9 je nach Durchmesser- bzw. Querschnittsverhältnis des jeweiligen Zylinderpaares. In der Regel sind die Verhältnisse so, dass bei gegebenem Stutzen-Achsabstand B und Zylinder-Achsabstand A die Stutzen 5 bei beiden Vorratszylindern eines Paares exzentrisch zur Zylinderachse 9 liegen; im Grenzfall (bei stark unterschiedlichen Zylinderdurchmessern innerhalb des Paares) kann es jedoch zweckmässig sein, am kleinen Zylinder die Stutzenachse 13 mit der Zylinderachse 9 zusammenfallen zu lassen und nur am grossen Zylinder den Stutzen entsprechend exzentrisch anzuordnen.

Ein gewichtiger Vorteil der beschriebenen, erfindungsgemässen Doppelkartuschen ist schliesslich in der Vermeidung von Umwelt-Kontamination zu sehen: Bei den eingangs erwähnten, bekannten Systemen mit Einzelkartuschen, die lösbar mit einem (wiederverwendbaren) Verbindungsteil zusammengefügt und nach Gebrauch wieder getrennt werden, können Restmengen von reaktiven, möglicherweise giftigen Materialkomponenten austreten. Dagegen wird die erfindungsgemässe Doppelkartusche samt Uebergangsteil und aufgestecktem Mischerrohr als Einheit beseitigt, die allseitig dicht verschlossen ist, nämlich vorn durch ausreagiertes Komponentengemisch im Mischerrohr und hinten durch die Förderkolben in den Zylindern. Somit können keine die Umwelt gefährdenden, reaktiven Restmengen austreten.

**Patentansprüche**

1. Doppel-Austragkartusche, die für die getrennte Langzeitlagerung und Verteilung von Zweikomponentenmassen von deren Abfüllung in die Doppel-Austragkartusche bis zur Verwendung durch den Verbraucher, sowie zur Bedienung der Kartusche mittels eines Austraggerätes beim Verbraucher bestimmt ist, mit zwei doppelwandig getrennten, miteinander verbundenen und parallelachsig nebeneinanderliegenden Vorratszylindern (2), die je eine stirnseitige Austrittsöffnung (4) aufweisen, wobei beide Austrittsöffnungen einander benachbart auf der gemeinsamen Durchmesserlinie (15) beider Vorratszylinder (2) liegen und wobei der Achsabstand (B) der Austrittsöffnungen (4) kleiner ist als der Achsabstand (A) der beiden Vorratszylinder (2), dadurch **gekennzeichnet,** dass ein den Vorratszylindern (2) vorgelagerter Uebergangsteil (20) als Bestandteil der Doppel-Austragkartusche (1) vorgesehen ist, welcher eine für beide Komponenten gemeinsame Mündung (22) zum Anschluss eines Mischerrohres (30) aufweist und zwei bis an das Ende der Mündung (22) getrennte Austragkanäle (23) enthält, und dass der Uebergangsteil (20) über eine permanente axiale Steckverbindung (5, 27, 6, 26), welche beide Austragkanäle (23) je an die Austrittsöffnung (4) der Vorratszylinder (2) anschliesst, mit beiden Vorratszylindern (2) verbunden ist, wodurch der Uebergangsteil (20) und beide Vorratszylinder (2) zusammen eine starre, zur getrennten Sangzeitlagerung beider Komponenten bestimmte Einheit (1) bilden.

2. Doppel-Austragkartusche nach Anspruch 1, dadurch gekennzeichnet, dass der als separater Spritzgussteil hergestellte Uebergangsteil (20) mittels je einer Schnappverbindung (6, 26) mit jedem der Vorratszylinder (2) verbunden ist.

3. Doppel-Austragkartusche nach Anspruch 1 oder 2, dadurch gekennzeichnet, dass die beiden Austragkanäle (23) im Uebergangsteil (20) auf ihrer ganzen Länge doppelwandig getrennt sind.

4. Doppel-Austragkartusche nach einem der Ansprüche 1 bis 3, dadurch gekennzeichnet, dass beide Austrittsöffnungen (4) je von einem kreisrunden Stutzen (5) umgeben sind und dass der Uebergangsteil (20) mit einer Anschlusspartie (21) die beiden Stutzen (5) übergreift.

5. Doppel-Austragkartusche nach einem der vorangehenden Ansprüche, dadurch gekennzeichnet, dass die beiden Austragkanäle (23) je durch zwei parallelachsig versetzte, einander überschneidende Längsabschnitte (24, 25) gebildet sind.

6. Doppel-Austragkartusche nach einem der vorangehenden Ansprüche, dadurch gekennzeichnet, dass die AusgangsQuerschnitte der beiden Austragkanäle (23) in der gemeinsamen Mündung (22) im gleichen Verhältnis zueinander bemessen sind wie die Querschnitte der beiden Vorratszylinder (2).

7. Doppel-Austragkartusche nach einem der vorangehenden Ansprüche, dadurch gekennzeichnet, dass die beiden Vorratszylinder (2) einstückig zusammenhängend sind.

8. Doppel-Austragkartusche nach Anspruch 7, dadurch gekennzeichnet, dass die beiden Vorratszylinder (2) im Bereich ihres hinteren und vorderen Endes durch Stege (8, 8') verbunden sind.

9. Doppel-Austragkartusche nach einem der Ansprüche 1 bis 6, dadurch gekennzeichnet, dass die beiden Vorratszylinder (2', 2") getrennt hergestellt und mindestens im Bereich ihres hinteren Endes durch eine distanzhaltende Steckoder Schnappverbindung (46) zusammengehalten sind.

10. Doppel-Austragkartusche nach Anspruch 9, dadurch gekennzeichnet, dass an den Vorratszylindern (2', 2"), vorzugsweise an ihrem Vorderende, eine Sicherung gegen Längsverschiebung vorgesehen ist.

11. Doppel-Austragkartusche nach Anspruch 4, dadurch gekennzeichnet, dass zwischen jedem Stutzen (5) und der Anschlusspartie (21) des Uebergangsteils eine O-Ring-Dichtung (7) vorgesehen ist.

12. Doppel-Austragkartusche nach einem der Ansprüche 1 bis 10, dadurch gekennzeichnet, dass jeder Stutzen (5) zur Anschlusspartie (21) des Uebergangsteils mittels einer Lippendichtung abgedichtet ist.

13. Doppel-Austragkartusche nach einem der vorangehenden Ansprüche, dadurch gekennzeichnet, dass die permanente Steckverbindung durch Kleben oder Schweissen gesichert ist.

14. Doppel-Austragkartusche nach Anspruch 4, dadurch gekennzeichnet, dass am Uebergangsteil (20) die Anschlusspartie (21) symmetrisch zur Achse (14) der kreisförmigen Querschnitt aufweisenden Mündung (22) gestaltet ist.

15. Doppel-Austragkartusche nach Anspruch 4, dadurch gekennzeichnet, dass an beiden Vorratszylindern (2) der Abstand (C) der Stutzenachse (13) zur Zylinder-Innenwand (12) gleich ist.

16. Baureihe von Doppel-Austragkartuschen mit gleichen Gesamt-Nennvolumen, aber verschiedenen Verhältnissen (1:1, 2:1, ... 10:1) zwischen den Querschnitten der Zylinderpaare, dadurch gekennzeichnet, dass der Zylinder-Achsabstand (A) bei allen Zylinderpaaren (2,2; 2a, 2b; ... 2g, 2h) der Baureihe gleich ist.

17. Baureihe nach Anspruch 16, dadurch gekennzeichnet, dass der Stutzen-Achsabstand (B) bei allen Zylinderpaaren der Baureihe gleich ist.

## Claims

1. Dual dispenser cartridge which is intended for the separate long-term storage and distribution of two-component compounds from their introduction into the dual dispenser cartridge to their use by the consumer and for operation of the cartridge by means of a dispensing device by the consumer, having two storage cylinders (2), which are separated by a double wall, are connected to one another, lie adjacent to one another with their axes parallel and each have a frontal dispensing opening (4), both dispensing openings lying adjacent to one another on the common diametral line (15) of both storage cylinders (2) and the centre-to-centre distance (B) of the dispensing openings (4) being smaller than the centre-to-centre distance (A) of the two storage cylinders (2), characterised in that a transition part (20) positioned in front of the storage cylinders (2) is provided as part of the dual dispenser cartridge (1), the said transition part having an orifice (22), common to both components, for the attachment of a mixer tube (30) and containing two dispensing channels (23) separated as far as the end of the orifice (22), and in that the transition part (20) is connected to both storage cylinders (2) via a permanent axial plug-in connection (5, 27, 6, 26) which connects each of the two dispensing channels (23) to the respective dispensing opening (4) of the storage cylinders (2), as a result of which the transition part (20) and both storage cylinders (2) together form a rigid unit (1) intended for the long-term storage of both components.

2. Dual dispenser cartridge according to Claim 1, characterised in that the transition part (20), manufactured as a separate injection-moulded component, is connected to each of the storage cylinders (2) by means of a respective snap-lock connection (6, 26).

3. Dual dispenser cartridge according to Claim 1 or 2, characterised in that the two dispensing channels (23) in the transition part (20) are separated by a double wall over their entire length.

4. Dual dispenser cartridge according to one of Claims 1 to 3, characterised in that each of the dispensing openings (4) is surrounded by a circular stub (5) and in that the transition part (20) engages over the two stubs (5) with a connecting region (21).

5. Dual dispenser cartridge according to one of the preceding claims, characterised in that each of the two dispensing channels (23) is formed by two mutually intersecting longitudinal portions (24, 25) offset parallel to the axis.

6. Dual dispenser cartridge according to one of the preceding claims, characterised in that the ratio of the dimensions of the exit cross-sections of the two dispensing channels (23) in the common orifice (22) to one another is the same as that of the cross-sections of the two storage cylinders (2).

7. Dual dispenser cartridge according to one of the preceding claims, characterised in that the two storage cylinders (2) are integrally joined.

8. Dual dispenser cartridge according to Claim 7, characterised in that the two storage cylinders (2) are connected by webs (8, 8') in the region of their rear and front ends.

9. Dual dispenser cartridge according to one of Claims 1 to 6, characterised in that the two storage cylinders (2', 2") are manufactured separately and held together at least in the region of their rear end by a spacing plug-in or snap-lock connection (46).

10. Dual dispenser cartridge as claimed in Claim 9, characterised in that a safeguard against longitudinal displacement is provided on the storage cylinders (2', 2"), preferably at their front end.

11. Dual dispenser cartridge according to Claim 4, characterised in that an O-ring seal (7) is provided between each stub (5) and the connecting region (21) of the transition part.

12. Dual dispenser cartridge according to one of Claims 1 to 10, characterised in that each stub (5) is sealed off in relation to the connecting region (21) of the transition part by means of a lip seal.

13. Dual dispenser cartridge according to one of the preceding claims, characterised in that the permanent plug-in connection is secured by bonding or welding.

14. Dual dispenser cartridge according to Claim 4, characterised in that the connecting region (21) on the transition part (20) is formed symmetrically to the axis (14) of the orifice (22), the latter having a circular cross-section.

15. Dual dispenser cartridge according to Claim 4, characterised in that the distance (C) of the stub axis (13) from the inner wall (12) of the cylinder is the same on both storage cylinders (2).

16. Series of dual dispenser cartridges with the same total nominal volumes but different ratios (1:1, 2:1, ... 10:1) between the cross-sections of the cylinder pairs, characterised in that the cylinder centre-to-centre distance (A) is the same for all the cylinder pairs (2, 2; 2a, 2b; ... 2g, 2h) of the series.

17. Series according to Claim 16, characterised in that the stub centre-to-centre distance (B) is the same for all the cylinder pairs of the series.

**Revendications**

1. Cartouche double, conçue pour le stockage de longue durée et la commercialisation sous une forme séparée des composants de mélanges à deux composants, depuis leur remplissage dans la cartouche double jusqu'à leur utilisation par le consommateur, et conçue pour être manipulée par le consommateur à l'aide d'un applicateur, comportant deux cylindres de stockage (2) séparés par une double paroi, mutuellement assemblés et juxtaposés avec des axes parallèles, qui présentent chacun une ouverture de sortie (4) du côté frontal, les deux ouvertures de sortie étant voisines sur la ligne diamétrale commune (15) des deux cylindres de stockage (2) et l'entraxe (B) des ouvertures de sortie (4) étant inférieur à l'entraxe (A) des cylindres de stockage (2),
**caractérisée** en ce que la cartouche double (1) comprend un élément réducteur (20) qui est monté devant les cylindres de stockage (2), présente une embouchure commune (22) pour les deux composants en vue du raccordement d'un tube mélangeur (30) et contient deux canaux d'expulsion séparés (23) à l'extrémité de l'embouchure (22), et en ce que l'élément réducteur (20) est assemblé aux deux cylindres de stockage (2) par un assemblage à emboîtement axial permanent (5, 27, 6, 26) qui raccorde respectivement les deux canaux d'expulsion (23) aux ouvertures de sortie (4) des cylindres de stockage (2), de sorte que l'élément réducteur (20) et les deux cylindres de stockage (2) forment conjointement un ensemble rigide (1), destiné au stockage séparé de longue durée des deux composants.

2. Cartouche double selon la revendication 1, caractérisée en ce que l'élément réducteur (20), réalisé sous la forme d'une pièce séparée moulée par injection, est assemblé à chacun des cylindres de stockage (2) par un assemblage à enclenchement respectif (6, 26).

3. Cartouche double selon la revendication 1 ou 2, caractérisée en ce que les deux canaux d'expulsion (23) de l'élément réducteur (20) sont séparés sur toute leur longueur par une double paroi.

4. Cartouche double selon l'une des revendications 1 à 3' caractérisée en ce que les deux ouvertures de sortie (4) sont chacune entourées par une tubulure circulaire (5), et en ce que l'élément réducteur (20) recouvre les deux tubulures (5) par une partie de raccordement (21).

5. Cartouche double selon l'une des revendications précédentes, caractérisée en ce que les deux canaux d'expulsion (23) sont chacun formés par deux tronçons longitudinaux (24, 25) d'axes parallèles décalés et qui se recoupent.

**6.** Cartouche double selon l'une des revendications précédentes, caractérisée en ce que les sections de sortie des deux canaux d'expulsion (23) dans l'embouchure commune (22) présentent le même rapport de dimensions que les sections des deux cylindres de stockage (2).

**7.** Cartouche double selon l'une des revendications précédentes, caractérisée en ce que les deux cylindres de stockage (2) sont contigus d'un seul tenant.

**8.** Cartouche double selon la revendication 7, caractérisée en ce que les deux cylindres de stockage (2) sont assemblés par des nervures (8, 8') dans la région de leur extrémité avant et arrière.

**9.** Cartouche double selon l'une des revendications 1 à 6, caractérisée en ce que les deux cylindres de stockage (2', 2") sont fabriqués séparément et réunis, au moins dans la région de leur extrémité arrière, par un assemblage écarteur à emboîtement ou enclenchement (46).

**10.** Cartouche double selon la revendication 9, caractérisée en ce qu'un moyen de blocage en translation longitudinale est prévu sur les cylindres de stockage (2', 2"), de préférence à leur extrémité avant.

**11.** Cartouche double selon la revendication 4, caractérisée en ce qu'un joint torique (7) est prévu entre chaque tubulure (5) et la partie de raccordement (21) de l'élément réducteur.

**12.** Cartouche double selon l'une des revendications 1 à 10, caractérisée en ce que chaque tubulure (5) est étanchée vers la partie de raccordement (21) de l'élément réducteur par un joint à lèvre.

**13.** Cartouche double selon l'une des revendications précédentes, caractérisée en ce que l'assemblage à emboîtement permanent est assujetti par collage ou soudage.

**14.** Cartouche double selon la revendication 4, caractérisée en ce que la partie de raccordement (21) est configurée sur l'élément réducteur (20) de manière symétrique à l'axe (14) de l'embouchure (22) pré sentant une section circulaire.

**15.** Cartouche double selon la revendication 4, caractérisée en ce que la distance (C) entre l'axe (13) de la tubulure et la paroi intérieure (12) du cylindre est la même sur les deux cylindres de stockage (2).

**16.** Gamme de fabrication de cartouches doubles présentant le même volume total mais des rapports différents (1:1, 2:1, ... 10:1) entre les sections des paires de cylindres, caractérisée en ce que l'entraxe (A) des cylindres est le même pour toutes les paires de cylindres (2,2; 2a, 2b; ... 2g, 2h) de la gamme.

**17.** Gamme de fabrication selon la revendication 16, caractérisée en ce que l'entraxe (B) des tubulures est le même pour toutes les paires de cylindres de la gamme.

Fig. 1

Fig. 2

Fig. 5

Fig. 6

Fig. 7

Fig. 3

Fig. 4